# EUROPEAN PATENT SPECIFICATION

(11) **EP 3 174 584 B1**
(45) Date of publication and mention of the grant of the patent: **04.05.2022**
(21) Application number: 15827303.7
(22) Date of filing: 29.07.2015
(51) Int. Cl.: A61M 11/00, A61M 16/00

(54) **POSITIVE PRESSURE INSPIRATION DEVICE FOR DELIVERY OF MEDICAMENTS**
POSITIVDRUCKINSPIRATIONSVORRICHTUNG ZUR ABGABE VON MEDIKAMENTEN
DISPOSITIF D'INSPIRATION À PRESSION POSITIVE PERMETTANT L'ADMINISTRATION DE MÉDICAMENTS

(30) Priority: 29.07.2014 US 201462030223 P
(43) Date of publication of application: 07.06.2017
(73) Proprietor: Edenhoffer, Peter, Paris, Texas 75460 (US)
(72) Inventor: Edenhoffer, Peter, Paris, Texas 75460 (US)
(74) Representative: Schwarz & Partner Patentanwälte GmbH
(86) International application number: PCT/US2015/042738
(87) International publication number: WO 2016/019061

(56) References cited:
- EP-A1- 2 119 465
- EP-A2- 2 537 548
- DE-A1- 19 939 417
- FR-A1- 2 799 978
- US-A- 2 693 178
- US-A- 5 479 920
- US-A1- 2004 134 494
- US-A1- 2007 227 535
- US-A1- 2013 327 323

## Description

### BACKGROUND

Degenerative processes which occur as a result of normal aging or disease processes can affect all humans and animals. The treatment of pathological and normal degenerative processes affecting different organs faces various obstacles such as accessing the organs and finding effective treatments.

Most organ systems can be accessed via the arterial and venous circulatory system. A less common route is via the nasopharyngeal and pulmonary system. Medications administered through the nasopharyngeal and pulmonary system may reach the nasal cavities, pharynx, larynx, trachea, bronchi, alveoli, and associated vascular and connective tissue structures.

In addition, in most animals, the nasal cavities provide a potential portal to the central nervous system through the olfactory nerves and associated vascular and connective tissue structures surrounding the olfactory bulb.

Currently, inhaled medications are administered into the nasopharyngeal passageways with simple sprays, inhalers, and nebulizers. These methods result in only partial absorption of the introduced materials into the body. Additionally, patients with significant pulmonary disease may have to exert great effort during the respiratory treatment, rendering the patient physically unable to complete the inhalation treatment.

Further, devices for administering medicaments to the nasopharyngeal passageways are typically configured to provide medicaments at a constant rate, resulting in waste of the medicaments. For example, nebulizers are typically configured to continuously channel and/or nebulize a source of medicament to a patient to be delivered to target treatment areas as the patient inspires. In such devices, however, a significant amount of the medicament is wasted. In particular, medicament is wasted during patient expiration, when medicament cannot be delivered to the patient, but is instead undesirably passed out of the device, passed into other areas of the device (becoming trapped in filters and/or potentially contaminating the medicament source), and/or left unused within the device (which also contributes to waste and potential contamination).

Some devices are configured to limit the amount of medicament channeled or nebulized during patient expiration. For example, some devices rely on electronic timers or mechanical baffles to try to align nebulization and delivery of medicament to a patient's inspiration/expiration cycle so as to only nebulize and deliver medicament during patient inspiration. However, the use of such devices still results in waste of the medicament. When inspiration ends, a residual amount of medicament that has already been nebulized and/or channeled toward the patient, but that has not yet been received by the patient, is left unused within the device or is undesirably passed out of the device upon subsequent patient expiration.

Nebulized medicament left unused within the device can also undesirably recoalesce within the device before subsequent patient inspiration, rendering the medicament undeliverable to the patient. For example, a significant amount of nebulized medicament remaining in the device at the end of patient inspiration will recoalesce from a fine mist into larger droplets or a liquid film, a form not capable of being delivered to a target nasopharyngeal area as intended.

Further, typical devices do not operate under a positive pressure, or only operate under positive pressure in conjunction with patient inspiration. Thus, the residual amount of medicament remaining in the device at a terminal phase of inspiration and/or at the end of inspiration is not subjected to the positive pressure that may be necessary to deliver the residual medicament to the patient.

In addition, techniques that rely on preset timers or lagging indicators (such as a previously measured breath cycle) cannot account for changes in a patient's breathing pattern from breath to breath. A significant portion of a patient's breathing can therefore be out of sync with the pre-determined medicament and/or gas delivery, resulting in wasted medicament and less efficient patient treatment.

The wasted amounts of medicament resulting from use of such devices and methods can be particularly costly when expensive medicaments, such as stem cells or platelets, are required as part of patient treatment. There has been and continues to be a need for devices and methods for efficient nasopharyngeal and pulmonary delivery of medicament with reduced waste of medicament. The document DE19939417 A1 discloses a nebuliser, which is operated by a pressure-sensitive switch that is responsive to two negative pressure thresholds to cause opening and closing of a valve. Thereby it is assured that medicaments are only nebulised during inhalation.

### BRIEF SUMMARY

The present disclosure is directed toward devices and methods for delivering medicament to the nasopharyngeal and/or pulmonary areas of a patient with eliminated or reduced waste of medicament.

One or more embodiments of the present disclosure relate to a respiratory system including a pressurized gas source coupled to a nebulizer containing a medicament, according to independent claim 1.

The claimed system allows residual medicament disposed within the system to be cleared out of the system and inhaled by the patient during the remainder of the inspiration cycle. A compressor can also be included to provide positive pressure and to aid in the delivery of the medicament to the patient.

The system of the present disclosure can improve the efficacy of treatments of degenerative processes. Certain embodiments can improve the efficiency by which medicaments, such as stem cells, platelets, growth factors, and/or cytokines are introduced into a person or animal. By administering these substances under positive pressure with materials which increase the permeability of cell membranes, and activating a nebulizer in synchrony with the appropriate portions of inspiration, these substances can be more efficiently introduced to the patient.

Administering medicaments through the nasal cavity can also allow the introduction of medications, stem cells, growth factors, and/or other trophic factors into the olfactory bulb. This can therefore provide access to the central nervous system and other organs outside the blood brain barrier through reverse axonal transport. In addition, administration of medicaments to the pulmonary system and associated circulatory systems can provide access to the respiratory system and associated vascular system through direct absorption.

Moreover, patients with and without respiratory disease have varying degrees of difficulty sustaining inspiration against resistance for prolonged periods of time and may become short of breath. With positive airway pressure, it is easier for patients to inspire, and therefore better patient compliance is likely to be achieved.

In some embodiments, the nebulizer can be activated at repeating intervals so that the medicament is nebulized at the beginning of the inspiratory cycle and then nebulization is stopped before the end of the inspiration phase of the respiratory cycle. This allows the remainder of the inspiratory breath to clear the chamber of nebulized material, which leaves minimal, if any, medicaments in the chamber unused. During expiration, the expiratory breath can be exhausted via a HEPA (high efficiency particulate air) filter.

The system of the present disclosure may be used to treat cystic fibrosis. In addition, one or more embodiments of the present disclosure are capable of providing precise quantities of medicament to be injected. This can be particularly beneficial, for example, with the use of stem cells, which can cause an inflammatory reaction at doses which are too high. In addition, delivery of medicaments to the olfactory bulb and/or olfactory nerves can beneficially deliver medicaments to the central nervous system of a patient. In some embodiments, administration under positive pressure (e.g., higher than ambient) can allow greater penetration of medicament to and into the olfactory bulb.

These and other advantages and features of the embodiments disclosed herein will become more fully apparent from the following description and appended claims.

### BRIEF DESCRIPTION OF THE DRAWINGS

To further clarify the above and other advantages and features of the present invention, a more particular description of the invention will be rendered by reference to specific embodiments thereof which are illustrated in the appended drawings. It is appreciated that these drawings depict only illustrated embodiments of the invention and are therefore not to be considered limiting of its scope. The invention will be described and explained with additional specificity and detail through the use of the accompanying drawings in which:
Figure 1 illustrates an embodiment of a respiratory system as disclosed in this application;
Figure 2 illustrates a close-up view of a nebulizer of the respiratory system of Figure 1, and associated components;
Figure 3 illustrates a side view of the respiratory system of Figure 1; and
Figure 4 illustrates the change in alveolar pressure over time during an inspiration phase of a typical respiratory cycle, showing a terminal portion of the inspiration in which residual medicament may be cleared from the respiratory system and delivered to a patient.

### DETAILED DESCRIPTION

### I. Introduction

One objective of the presently disclosed respiratory system is to deliver medicaments under positive pressure and at metered doses so as to prevent waste of the medicament. The medicament delivered by a nebulizer may include any medicament capable of being nebulized, including but not limited to, platelet rich plasma, stem cells, growth factors, cytokines, hyaluronidase, and other medications. Target organs and/or organ systems can include the olfactory bulbs, pulmonary system, and associated vascular systems. The system of the present disclosure may be used to both treat a disease state as well as to rejuvenate and improve functioning of the pulmonary system, olfactory system, or nasopharyngeal tissues.

Treatment duration may vary from a few minutes to over an hour at a time. Treatments may initially be repeated on a daily basis or less frequently, and may be repeated at intervals during the year, depending on the underlying process being treated and degree of response of the patient.

Outcomes are measured based on physiological testing, such as pulmonary function test, physical endurance, as well as a subjective sense of improved endurance. When treating the central nervous system, imaging studies may be used to monitor progress and cognitive functioning testing may be performed.

The apparatus of the disclosure may also be used for administering pharmaceutical medications, synthetic materials, and chemotherapy medications. Essentially any solution may be introduced with the apparatus.

### II. Exemplary Medicaments

Primary healing and regeneration can be achieved by the introduction of stem cells or platelets which are either intact or lysed. They may be introduced with associated growth factors and cytokines. The described embodiments, in contrast to currently available techniques, can decrease waste of nebulized material, improve absorption, and eases the patient's workload, and therefore may improve efficacy of treatment and patient compliance with treatment.

Increased penetration of medicaments through increased cell permeability can be achieved with medications such as hyaluronidase. Preferred embodiments can include administering the medicaments under positive airway pressure, which can increase the transmural pressure and therefore absorption of the medicaments. The effectiveness of hyaluronidase can be increased by administering at neutral pH levels, which may be achieved by using a phosphate buffer saline or other buffered solutions. Such pH neutral hyaluronidase can then be used to pretreat the biological absorptive surface prior to delivering stem cells and/or other factors. Alternatively, stem cells and/or other factors may be suspended in hyaluronidase and then delivered. Various concentrations may be used. For example, suspending cells in about 5 to 45 micrograms, or about 10 to 40 micrograms, or about 15 to 30 micrograms, or about 20 to 25 micrograms of a solution including hyaluronidase at a concentration ranging from about 1 to 20 mg/ml, or about 5 to 15 mg/ml, or about 10 mg/ml. In some embodiments, about 20 micrograms of a stem cell medicament (which may include cytokines and/or other factors) is suspended in a solution including hyaluronidase at a concentration of about 10 mg/ml.

Platelet rich plasma and growth factors can be obtained from blood plasma. Stem cells may be obtained from mesenchymal stem cells in adipose tissue, from bone marrow aspirate, and/or from other sources. The samples may be obtained following standard protocols for obtaining such samples. After collection, cells may be administered immediately after purification and concentration, or after further cell line expansion to increase cell load to be injected.

For example, in the case of platelet rich plasma and growth factors, blood samples may be taken and centrifuged, with the desired components aspirated from the centrifuged samples. The aspirated components can then be pooled and placed into the nebulizer.

Stem cells can be obtained from adipose tissue and/or bone marrow with standard harvesting techniques, followed by washing and resuspension. Viability of the harvested material is ensured by the use of fresh material which is reintroduced into the body within two hours of harvesting. Other methods of preserving the harvested material include refrigeration, freezing, and incubation with cell line expansion. Freezing of cells results in the production of lysate which may further improve efficacy of treatment.

The vehicle for delivery of the medicament varies depending on the type of medicament that is used for the treatment and the delivery target. It may be necessary or desirable to suspend or mix the treatment material in a carrier or other pharmaceutically acceptable substance. For platelet rich plasma, the plasma itself can act as a carrier but may be supplemented with other materials so as to change viscosity and/or increase cell penetration. Stem cells may be suspended in saline. Cell suspension and efficacy may be improved with the use of pH neutral solutions and optimized hyaluronidase concentrations.

### III. Delivery Devices

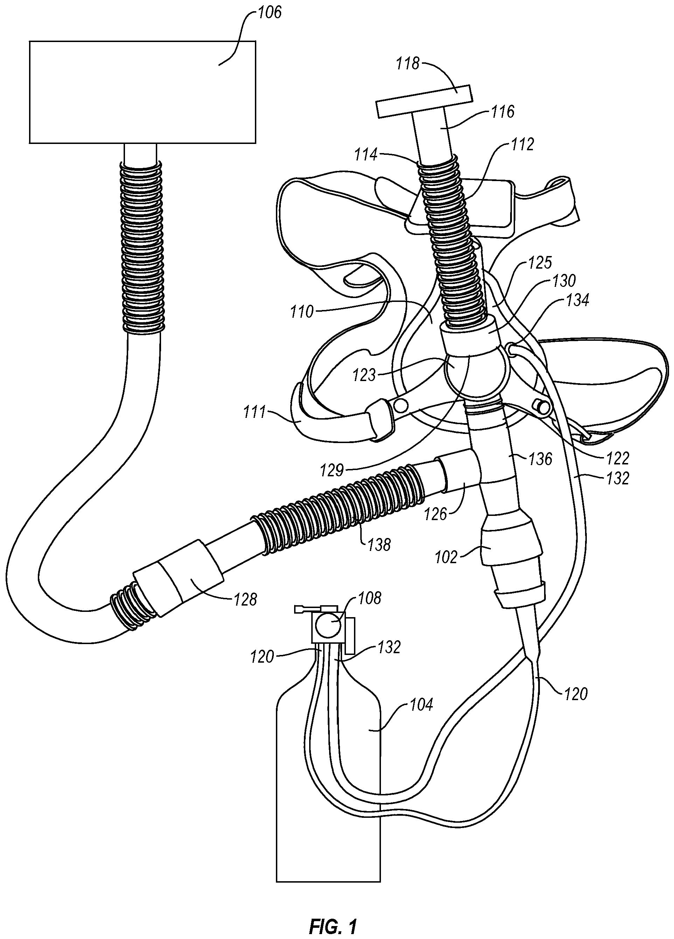

Figure 1 illustrates an exemplary embodiment of a respiratory system. Figure 1 shows a nebulizer 102 which can be propelled by tank 104 containing compressed oxygen or air. Airflow pressure from the nebulizer 102 can be increased by a compressor 106 (such as a Biphasic Positive Airway Pressure (BiPAP^{®}) or other positive pressure components, which can have variable pressure and can be modulated. Flow from tank 104 may be controlled by a negative pressure sensitive switch 108, activated by negative pressure generated in a breathing unit, illustrated in this embodiment as breathing mask 110.

The nebulizer 102 is preferably a jet nebulizer configured to provide a fine mist of nebulized medicament in response to the passage of compressed air through the nebulizer 102. Such jet nebulizers can beneficially allow the use of medicaments including larger proteins, cells, and cell components. In other embodiments, the nebulizer 102 may be formed as an ultrasonic nebulizer or other form of nebulizer.

As used herein, the terms "nebulize," "nebulizer," "nebulized," and the like relate to the production of mist to be delivered to the nasopharyngeal and/or pulmonary tissues of a patient. As used herein, such terms are synonymous with "aerosol," "aerosolizer," "aerosolized," "inhaler," "inhalant," and the like.

An exhaust manifold 112 may be connected to the mask 110 and can contain at its distal end 114 a one-way valve 116 and filter 118 (e.g., a high-efficiency particulate arrestance ("HEPA") filter). Breathing mask 110 as shown can be designed to fit over a patient's nose and/or mouth, and can have straps 111 or other attachment means to help fix the mask 110 in position on a patient. In other embodiments, breathing mask 110 may alternatively be configured as a nasal pillow mask, a nasal mask, a mouth-only mask or tube, or other type of delivery structure configured to enable delivery of air and/or medicament to a patient's nasopharyngeal and/or pulmonary systems.

Oxygen, air, or other gas can flow from tank 104 to nebulizer 102 through a gas line 120. Airflow direction can be controlled by negative pressure generated as well as positive pressure generated by the compressor 106. Several valves (e.g., one-way valves) throughout the system can ensure proper airflow. Valve 122, which can be configured as a lower pressure valve (e.g., is actuated at lower pressures relative to other valves of the device), can be located in the mask 110. Valve 122 can serve to connect the compressor 106 and nebulizer 102 to the breathing mask 110. Medicaments and/or gas can flow from the nebulizer 102 and compressor 106 through valve 122 and into the breathing mask 110. Valve 122 can be configured to close prior to the opening of valve 116 (e.g., by configuring valve 122 to actuate at a lower pressure threshold relative to valve 116), thereby ensuring that the patient's expiratory volume exits through the exhaust manifold 112.

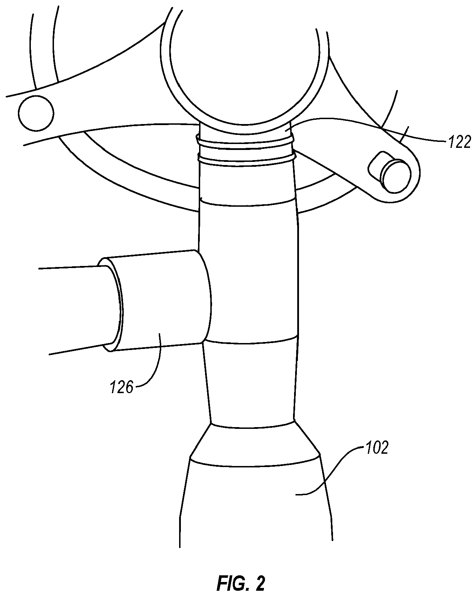

Figure 2 is a close-up view of the nebulizer 102 and nearby components of the system 100 illustrated in Figure 1. The nebulizer 102 in this embodiment can be powered by compressed oxygen and/or compressed air (e.g., delivered through tank 104, pressurized gas line, or other compressed gas delivery means).

Referring back to Figure 1, the nebulizer 102 can be activated by a negative pressure sensitive switch 108, which may be disposed on tank 104 as illustrated or at other sections of the device providing access to the gas line 120. Face mask 110 can be connected to a trigger hose 132 (e.g., via trigger port 134) which is also in communication with the pressure sensitive switch 108. When a patient wearing mask 110 inspires, the pressure in the trigger hose 132 can drop and can trigger the pressure sensitive switch 108. In the illustrated embodiment, when the switch 108 is activated by negative pressure during patient inspiration, airflow is provided to the nebulizer 102.

Medicament, such as stem cells, platelet rich plasma, other medicaments described herein, or other medicaments useful for nasopharyngeal and/or pulmonary delivery, can be located in the nebulizer 102. Such medicament may be positioned in the nebulizer 102 before the system 100 is assembled or may be injected or otherwise positioned into the nebulizer after assembly is partially or totally completed.

The medicament in the nebulizer 102 can be aerosolized by the gas from the tank 104, and the nebulized particles can travel through the device toward and then through the valve 122 to be inhaled by a patient wearing mask 110. The valve 122 may connect to an inlet port 123 that allows the passage of gas and medicament between an outer surface 125 of the face mask and an inner surface 127 of the face mask.

Compressed air flow may also be triggered manually to adjust the timing of the delivery of nebulized medicament during the initial portion of the inspiratory phase. For example, the system 100 can include a manual trigger actuator configured such that a patient or caretaker can manually actuate (via button, knob, switch, etc.) the pressure sensitive switch 108 in order to initiate compressed air flow and nebulization of the medicament within the nebulizer 102.

After actuation, the manual trigger actuator is configured to maintain automatic closing of pressure sensitive switch 108 when the amount of negative pressure lessens to below a threshold level. As explained in more detail below, such functionality allows residual medicament remaining in the device to be cleared from the device and delivered to the patient during the terminal portion of the inspiration phase.

In some embodiments, a controllable valve, such as a solenoid valve (e.g., in conjunction with a programmable regulator) may be used to stop the flow of nebulized material prior to the end of inspiration, thus allowing for inspiration of all material in the inspiration mask, thereby reducing waste of medicament. For example, the valve can be programmed to stop the flow of gas according to a preset time. In preferred embodiments, however, gas flow and nebulization of medicament is stopped based on real-time determination of inspiration parameters, as described in more detail below.

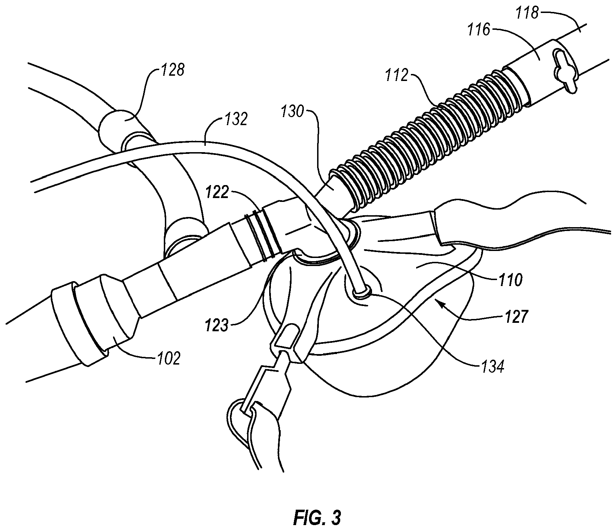

Figure 3 illustrates another view of the system 100 showing the placement of the valves 128, 126, 122, 116, and 130. Valve 122 can be positioned so as to prevent the backflow (toward the nebulizer 102) of expired breath during expiration. The closure of the valve 122 during expiration thus forces air to travel into exhaust manifold 112 and out through one-way valve 116. Valve 130 can be positioned so as to prevent passage of medicament-containing air into the exhaust manifold 112 during inspiration. Valve 130 may be controlled by manual switching and/or may be automatically actuated according to appropriate pressure changes (e.g., closes at lower pressures during inspiration and opens at higher pressures during expiration). In some embodiments, the valve 130 may connect to an outlet port 129 (see Figure 1) that allows the passage of gas and/or medicament between an outer surface 125 of the face mask 110 and an inner surface 127 of the face mask 110 (e.g., for a small portion of gas and/or medicament not first passing through inlet port 123). Valves 126 and 128 can control and/or channel gas flow from the compressor 106 (not shown in Figure 2). For example, valves 126 and/or 128 can prevent backflow (flow toward the compressor) of air or gas directed toward the patient.

Referring to Figure 1, the connection conduit 136 between the nebulizer 102 and the inlet port 123 may be T-shaped. The connection conduit 136 may connect to valve 126, which may connect to line 138, which may connect to the compressor 106 (e.g., a Biphasic Positive Airway Pressure (BiPAP^{®}) compressor).

### IV. Efficient Delivery of Medicament

One or more embodiments of the present disclosure can improve the efficiency by which stem cells, platelets, growth factors, cytokines, and/or other medications are introduced into a person or animal. For example, by administering these medicaments under positive pressure (e.g., as a result of the compressor 106), and activating a nebulizer 102 during the appropriate portions of the inspiratory cycle, these medicaments can be more efficiently introduced.

Moreover, patients with and without respiratory disease have varying degrees of difficulty sustaining inspiration against resistance for prolonged periods of time and may become short of breath. With positive airway pressure, it is easier for patients to inspire, and therefore better patient compliance is likely to be achieved.

In some embodiments, nebulizer 102 can be actuated intermittently so that the medicament is nebulized at the beginning of the inspiratory cycle and then nebulization is stopped before the end of the respiratory cycle. This allows the residual amount of medicament to be cleared from the device as it is delivered to the patient during a terminal phase of inspiration (e.g., the portion of inspiration following cessation of nebulization). Such a configuration can leave minimal, if any, medicament in the lines, tubing, valves, mask 110 (e.g., the space between the inside surface of mask 110 and the patient's face), and other portions of the device unused.

In some embodiments, the pressure sensitive switch 108 can be a pressure regulated switch (e.g., electrical, mechanical, or electromechanical) configured to allow adjustment of one or more pressures at which the switch will open and/or close. For example, the pressure sensitive switch 108 can be attached to a control circuit that is programmable to provide selection and adjustment of an actuation pressure and/or a cessation pressure.

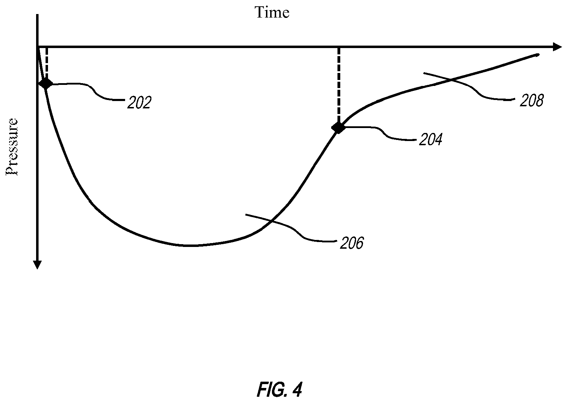

Figure 4 schematically illustrates alveolar pressure change over time during a typical inspiration portion of a respiratory cycle. As illustrated, a pressure sensitive switch is configured to actuate (so as to provide nebulization and delivery of medicament) upon reaching a first threshold pressure 202. The pressure sensitive switch is also configured to close (so as to prevent further nebulization and delivery of medicament) upon reaching a second threshold pressure 204. The time period 206 indicates the time during which medicament is being nebulized and delivered to the patient. Time period 208 indicates the terminal portion of the inspiration phase. In this period, residual medicament residing within the device (e.g., within the tubing, lines, valves, mask, and/or space between the mask and face) is partially or fully cleared from the device and delivered to the patient as intended. In addition, in some embodiments, continued positive pressure (e.g., from a compressor such as a BiPAP^{®} compressor) further directs residual medicament and gas volume into the nasopharyngeal and/or pulmonary areas of the patient to deliver the medicament and prevent wasted medicament.

In other embodiments, threshold pressures 202 and/or 204 can be adjusted according to patient and/or care provider needs and preferences. For example, first threshold pressure 202 can be adjusted to provide reliable initiation of nebulization and gas flow and/or second threshold pressure 204 can be adjusted to provide an adequate balance between time period 206 in which nebulization is active and time period 208 in which medicament is cleared.

In addition, during expiration, the expiratory breath can exhausted via filter 118 (e.g., HEPA filter). Filter 118 can be configured to prevent uninspired stem cells or other materials (if any) from exiting the system.

Embodiments of the present disclosure can provide a number of benefits. For example, the system can be configured to prevent nebulization during expiration, during resting periods between breaths, and during a terminal portion of the inspiration phase. In addition, the system does not rely on preconfigured timing sequences attempting to match nebulization and the breathing cycle to a consistent pattern. For example, the device can operate to deliver nebulized medicament while still clearing the device to prevent waste even if the patient has an inconsistent or erratic breathing pattern, such as a short, shallow breath followed by a deep, long breath, or vice versa. Further, the device does not rely on lagging indicators (such as one or more of the previous breath cycles) to estimate current breath activity, but instead operates in real-time to deliver medicament during inspiration without over-providing medicament so as to lead to waste.

Moreover, the actuation and cessation of nebulization does not require monitoring of the compressor or the determination of compressor-related parameters. For example, the negative pressure sensitive switch can be opened and/or closed according to criteria independent from the compressor.

### V. Olfactory Bulb Administration

In some embodiments, the device can be directed toward the olfactory epithelium and bulb. This can be accomplished by adjusting the positive airway pressure to a variable frequency pulsing at about 2 to 200 Hz, or about 25 to 150 Hz, or about 50 to 100 Hz. In some embodiments, the face mask 110 can include a nasal adaptor configured to direct nebulized medicament to the cribriform plate of the patient. For example, the nasal adaptor can include sealed prongs to seal the nasal passageways and variable length extension tubes directed to the cribriform plate. In this configuration, positive pressure provided at variable pulse frequency and/or velocity can enhance flow to the cribriform plate of the patient.

In some embodiments, the nasal adaptor of the face mask 110 can include a first sealed prong for positioning in a first nostril of a patient, and a variable length extension extending from the first sealed prong toward the cribriform plate. The second nostril can be sealed with a second prong including a one way valve configured to allow outflow of gas and nebulized medicament introduced through the first nostril. Positive pressure may also be provided at variable velocity and/or pulse frequencies to enhance flow to the cribriform plate.

In some embodiments, the face mask 110 may also include an expiratory tube insertable into a patient's mouth. The expiratory tube may enable a patient to blow air through and breathe through the tube so as to conscientiously close the soft palate. Embodiments utilizing such an expiratory tube may be useful in circumstances where it is desired or required that nebulized medicament be passed to the olfactory bulb and not the pulmonary system. In addition, closure of the soft palate can be monitored by measuring the amount of pressure the patient exhales into the expiratory tube. For example, a pressure sensitive solenoid switch can be coupled to the gas flow, and can be configured to cut off gas flow to the nasal cavity upon sensing a pressure drop during patient expiration (e.g., drops below a threshold value during an otherwise normal expiration phase of a respiratory cycle). For example, the present invention may be modified for use with a ventilator and an endotracheal tube that is inserted through the mouth or nose (i.e., intubation), or through a breathing tube placed through the front of the neck via a tracheostomy. The described embodiments are to be considered in all respects only as illustrative and not restrictive. The scope of the invention is, therefore, defined by the appended claims rather than by the foregoing description. All changes which come within the meaning of the claims are to be embraced within their scope.

## Claims

1. A respiratory system for delivery of a medicament to a patient, comprising:
a pressurized gas source (104);
a nebulizer (102) containing a medicament, the nebulizer being configured to receive pressurized gas from the pressurized gas source to nebulize at least a portion of the medicament, and to direct nebulized medicament toward a breathing unit (110) configured to deliver nebulized medicament to the patient; and
a pressure sensitive switch (108) associated with the pressurized gas source and the breathing unit, wherein the pressure sensitive switch is configured to open upon a first threshold negative pressure level to release pressurized gas, and to close upon a second threshold negative pressure level, the first and second threshold levels occurring during an inspiratory phase of a respiratory cycle,
wherein the second threshold negative pressure level is set at a lower pressure level relative to the first threshold negative pressure level such that when the breathing unit is used by a patient, the pressure sensitive switch closes before an ending of an inspiratory phase of a respiratory cycle of the patient,
wherein the first threshold negative pressure level is set at a level that occurs at an initial portion of an inspiratory phase of a respiratory cycle and the second threshold negative pressure is set at a level that occurs upon reaching a terminal portion of the inspiratory phase of the respiratory cycle.

2. The respiratory system of claim 1, **characterized in that** the pressure sensitive switch is connected to the breathing unit by a trigger hose (132), the trigger hose enabling negative pressure during inspiration to be detected by the pressure sensitive switch.

3. The respiratory system of claim 1 or claim 2, **characterized in that** the medicament includes one or more of stem cells, platelets, growth factors, cytokines, or hyaluronidase.

4. The respiratory system of any one of claims 1 to 3, **characterized in that** the medicament includes a suspension of 5 to 45 micrograms of cells in a solution having a hyaluronidase concentration of 1 to 20 mg/ml, and preferably where the medicament is at a substantially neutral pH.

5. The respiratory system of any one of claims 1 to 4, further comprising a compressor (106) in fluid communication with the breathing unit, the compressor configured to provide positive air pressure to the breathing unit.

6. The respiratory system of claim 5, **characterized in that** the compressor is coupled to the breathing unit at a location between the nebulizer and the breathing unit.

7. The respiratory system of claim 5 or claim 6, **characterized in that** the compressor is disposed proximal to the nebulizer and the pressurized gas source is disposed distal to the nebulizer.

8. The respiratory system of any one of claims 1 to 7, further comprising a one-way valve (122) disposed between the nebulizer and the breathing unit and configured to prevent backflow of gas toward the nebulizer.

9. The respiratory system of any one of claims 1 to 8, further comprising an exhaust manifold (112) coupled to the breathing unit, the exhaust manifold including a one-way valve (130) configured to prevent flow of nebulized medicament into the exhaust manifold during inspiration.

10. The respiratory system of any one of claims 1 to 9, **characterized in that** the breathing unit includes a nasal adaptor configured to deliver a medicament toward the cribriform plate of the patient.

11. The respiratory system of claim 10, further comprising a compressor (106) configured to provide positive pressure to the nasal adaptor at a variable frequency of about 2 to 200 Hz.

## Patentansprüche

1. Respirationssystem zur Abgabe eines Medikaments an einen Patienten, umfassend:
eine Druckgasquelle (104);
einen Vernebler (102), der ein Medikament enthält, wobei der Vernebler konfiguriert ist, um Druckgas von der Druckgasquelle zu empfangen, um zumindest einen Teil des Medikaments zu vernebeln und um das vernebelte Medikament zu einer Atmungseinheit (110) zu leiten, die konfiguriert ist, um das vernebelte Medikament an den Patienten abzugeben;und
einen druckempfindlichen Schalter (108), welcher der Druckgasquelle und der Atmungseinheit zugeordnet ist, wobei der druckempfindliche Schalter konfiguriert ist, um sich bei einem ersten Unterdruck-Schwellenniveau zu öffnen, um Druckgas abzugeben, und sich bei einem zweiten Unterdruck-Schwellenniveau zu schließen, wobei das erste und das zweite Schwellenniveau während einer Inspirationsphase eines Atemzyklus auftreten,
wobei das zweite Unterdruck-Schwellenniveau in Bezug auf das erste Unterdruck-Schwellenniveau auf ein niedrigeres Druckniveau eingestellt ist, sodass sich der druckempfindliche Schalter bei Verwendung der Atmungseinheit durch einen Patienten vor dem Ende einer Inspirationsphase eines Atemzyklus des Patienten schließt,
wobei das erste Unterdruck-Schwellenniveau auf ein Niveau eingestellt ist, das in einem anfänglichen Abschnitt einer Inspirationsphase eines Atemzyklus auftritt, und das zweite Unterdruck-Schwellenniveau auf ein Niveau eingestellt ist, das beim Erreichen eines Endabschnitts der Inspirationsphase des Atemzyklus auftritt.

2. Respirationssystem nach Anspruch 1, **dadurch gekennzeichnet, dass** der druckempfindliche Schalter durch einen Auslöseschlauch (132) mit der Atmungseinheit verbunden ist, wobei der Auslöseschlauch die Detektion eines Unterdrucks während der Inspiration durch den druckempfindlichen Schalter ermöglicht.

3. Respirationssystem nach Anspruch 1 oder Anspruch 2, **dadurch gekennzeichnet, dass** das Medikament eines oder mehrere aus Stammzellen, Thrombozyten, Wachstumsfaktoren, Zytokinen oder Hyaluronidase umfasst.

4. Respirationssystem nach einem der Ansprüche 1 bis 3, **dadurch gekennzeichnet, dass** das Medikament eine Suspension von 5 bis 45 µg Zellen in einer Lösung mit einer Hyaluronidase-Konzentration von 1 bis 20 mg/ml umfasst, wobei das Medikament vorzugsweise einen im Wesentlichen neutralen pH aufweist.

5. Respirationssystem nach einem der Ansprüche 1 bis 4, das außerdem einen Kompressor (106) in Fluidkommunikation mit der Atmungseinheit umfasst, wobei der Kompressor konfiguriert ist, um Luftüberdruck an die Atmungseinheit bereitzustellen.

6. Respirationssystem nach Anspruch 5, **dadurch gekennzeichnet, dass** der Kompressor mit der Atmungseinheit an einer Stelle zwischen dem Vernebler und der Atmungseinheit gekoppelt ist.

7. Respirationssystem nach Anspruch 5 oder Anspruch 6, **dadurch gekennzeichnet, dass** der Kompressor proximal zum Vernebler angeordnet ist und die Druckgasquelle distal zum Vernebler angeordnet ist.

8. Respirationssystem nach einem der Ansprüche 1 bis 7, das außerdem ein Einwegventil (122) umfasst, das zwischen dem Vernebler und der Atmungseinheit angeordnet ist und konfiguriert ist, um Rückströmung von Gas zum Vernebler hin zu verhindern.

9. Respirationssystem nach einem der Ansprüche 1 bis 8, das außerdem einen Abgassammler (112) umfasst, der mit der Atmungseinheit verbunden ist, wobei der Abgassammler ein Einwegventil (130) umfasst, das konfiguriert ist, um das Strömen von vernebeltem Medikament in den Abgassammler während der Inspiration zu verhindern.

10. Respirationssystem nach einem der Ansprüche 1 bis 9, **dadurch gekennzeichnet, dass** die Atmungseinheit einen Nasenadapter umfasst, der konfiguriert ist, um ein Medikament zur Siebplatte des Patienten hin abzugeben.

11. Respirationssystem nach Anspruch 10, das außerdem einen Kompressor (106) umfasst, der konfiguriert ist, um Überdruck mit einer variablen Frequenz von etwa 2 bis 200 Hz zur Siebplatte des Patienten bereitzustellen.

## Revendications

1. Système respiratoire pour l'administration d'un médicament à un patient, comprenant :
une source de gaz sous pression (104) ;
un nébuliseur (102) contenant un médicament, le nébuliseur étant configuré pour recevoir du gaz sous pression en provenance de la source de gaz sous pression pour nébuliser au moins une partie du médicament, et pour diriger le médicament nébulisé vers une unité de respiration (110) configurée pour administrer le médicament nébulisé au patient ; et
un interrupteur sensible à la pression (108) associé à la source de gaz sous pression et à l'unité de respiration, dans lequel l'interrupteur sensible à la pression est configuré pour s'ouvrir lors d'un premier niveau de pression négative seuil pour libérer du gaz sous pression, et pour se fermer lors d'un second niveau de pression négative seuil, les premier et second niveaux seuil se produisant pendant une phase inspiratoire d'un cycle respiratoire,
dans lequel le second niveau de pression négative seuil est établi à un niveau de pression inférieur par rapport au premier niveau de pression négative seuil de sorte que, lorsque l'unité de respiration est utilisée par un patient, l'interrupteur sensible à la pression se ferme avant une fin d'une phase inspiratoire d'un cycle respiratoire du patient,
dans lequel le premier niveau de pression négative seuil est établi à un niveau qui se produit à une partie initiale d'une phase inspiratoire d'un cycle respiratoire et le second niveau de pression négative seuil est établi à un niveau qui se produit lorsqu'une partie terminale de la phase inspiratoire du cycle respiratoire est atteinte.

2. Système respiratoire selon la revendication 1, **caractérisé en ce que** l'interrupteur sensible à la pression est relié à l'unité de respiration par un tuyau de déclenchement (132), le tuyau de déclenchement permettant à une pression négative pendant l'inspiration d'être détectée par l'interrupteur sensible à la pression.

3. Système respiratoire selon la revendication 1 ou la revendication 2, **caractérisé en ce que** le médicament comprend un ou plusieurs parmi des cellules souches, plaquettes, facteurs de croissance, cytokines, ou hyaluronidase.

4. Système respiratoire selon l'une quelconque des revendications 1 à 3, **caractérisé en ce que** le médicament comprend une suspension de 5 à 45 microgrammes de cellules dans une solution ayant une concentration en hyaluronidase de 1 à 20 mg/ml, et de préférence où le médicament est à un pH sensiblement neutre.

5. Système respiratoire selon l'une quelconque des revendications 1 à 4, comprenant en outre un compresseur (106) en communication fluidique avec l'unité de respiration, le compresseur étant configuré pour fournir une pression d'air positive à l'unité de respiration.

6. Système respiratoire selon la revendication 5, **caractérisé en ce que** le compresseur est couplé à l'unité de respiration à un emplacement entre le nébuliseur et l'unité de respiration.

7. Système respiratoire selon la revendication 5 ou la revendication 6, **caractérisé en ce que** le compresseur est disposée da manière proximale par rapport au nébuliseur et la source de gaz sous pression est disposée de manière distale par rapport au nébuliseur.

8. Système respiratoire selon l'une quelconque des revendications 1 à 7, comprenant en outre une valve unidirectionnelle (122) disposée entre le nébuliseur et l'unité de respiration et configurée pour empêcher un reflux de gaz vers le nébuliseur.

9. Système respiratoire selon l'une quelconque des revendications 1 à 8, comprenant en outre un collecteur d'échappement (112) couplé à l'unité de respiration, le collecteur d'échappement comprenant une valve unidirectionnelle (130) configurée pour empêcher un écoulement de médicament nébulisé dans le collecteur d'échappement pendant l'inspiration.

10. Système respiratoire selon l'une quelconque des revendications 1 à 9, **caractérisé en ce que** l'unité de respiration comprend un adaptateur nasal configuré pour administrer un médicament en direction de la lame criblée du patient.

11. Système respiratoire selon la revendication 10, comprenant en outre un compresseur (106) configuré pour fournir une pression positive à l'adaptateur nasal à une fréquence variable d'environ 2 à 200 Hz.
